(19) Europäisches Patentamt European Patent Office Office européen des brevets

(11) **EP 4 286 049 A1**

(12) **EUROPEAN PATENT APPLICATION**
published in accordance with Art. 153(4) EPC

(43) Date of publication:
06.12.2023 Bulletin 2023/49

(21) Application number: 22745945.0

(22) Date of filing: 26.01.2022

(51) International Patent Classification (IPC):
*B01J 27/199* (2006.01)    *C07B 61/00* (2006.01)
*C07C 51/235* (2006.01)    *C07C 57/055* (2006.01)

(52) Cooperative Patent Classification (CPC):
**B01J 27/199; C07B 61/00; C07C 51/235; C07C 57/04**

(86) International application number:
**PCT/JP2022/002960**

(87) International publication number:
**WO 2022/163726 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 27.01.2021 JP 2021010889

(71) Applicant: **Nippon Kayaku Kabushiki Kaisha Tokyo 100-0005 (JP)**

(72) Inventors:
• **OBATA, Tomohiro**
**Sanyoonoda-shi, Yamaguchi 757-8686 (JP)**
• **KAWAGUCHI, Toru**
**Sanyoonoda-shi, Yamaguchi 757-8686 (JP)**
• **HIRAOKA, Ryota**
**Sanyoonoda-shi, Yamaguchi 757-8686 (JP)**
• **SAKAI, Hideomi**
**Sanyoonoda-shi, Yamaguchi 757-8686 (JP)**

(74) Representative: **Gille Hrabal**
**Partnerschaftsgesellschaft mbB**
**Patentanwälte**
**Brucknerstraße 20**
**40593 Düsseldorf (DE)**

(54) **CATALYST, AND METHOD FOR PRODUCING UNSATURATED CAROBXYLIC ACID USING SAME**

(57) A catalyst contains molybdenum, copper, and vanadium as essential components, wherein a ratio (X/Y) of a pore volume (X) of pores in a range of a pore diameter from 25 $\mu$m to 400 $\mu$m to a pore volume (Y) of pores in a range of a pore diameter from 0.1 $\mu$m to 4 $\mu$m, measured by mercury porosimetry, is 1.10 or more and 3.00 or less.

EP 4 286 049 A1

**Description**

TECHNICAL FIELD

The present invention relates to a catalyst for obtaining an unsaturated carboxylic acid by an oxidation reaction, and to a catalyst with which a desired product can be obtained with higher selectivity than that of catalysts in the related art.

BACKGROUND ART

Numerous catalysts have been proposed as catalysts for use in producing unsaturated carboxylic acids. A catalyst for producing methacrylic acid, among the unsaturated carboxylic acids, contains molybdenum and phosphorus as main components and has a structure of heteropoly acid and/or salt. In addition, many proposals have been made for methods for producing these catalysts.

Many proposals have been made so far for catalysts for producing methacrylic acid. One of them is a method in which a catalyst fine structure such as a pore volume and a pore distribution is prepared by a catalyst shaping method or shaping condition. For example, Patent Literature 1 proposes a catalyst for producing methacrylic acid, in which a proportion of a pore volume of pores having a pore diameter radius of 0.005 $\mu$m to 0.05 $\mu$m with respect to a pore volume of pores having a pore diameter radius of 0.005 $\mu$m to 10 $\mu$m is 20% or more.

Patent Literature 2 proposes a catalyst for producing methacrylic acid, in which a pore volume of the catalyst is 0.1 cc/g or more and 0.9 cc/g or less, a pore volume occupied by pores having a pore diameter of less than 0.1 $\mu$m is 15% or less of a total pore volume, a pore volume occupied by pores having a pore diameter of 0.1 $\mu$m or more and less than 1 $\mu$m is less than 15% of the total pore volume, and a pore volume occupied by pores having a pore diameter of 1 $\mu$m or more and less than 10 $\mu$m is 65% or more of the total pore volume. Patent Literature 3 proposes a catalyst for producing methacrylic acid, in which a pore volume in a pore radius of 0.05 $\mu$m or less is 0.01 cc/g or less, and a pore volume in a pore radius of more than 0.05 $\mu$m is 0.2 cc/g or more.

However, the catalysts obtained as in Patent Literatures 1 to 3 have problems such as low desired product selectivity, short life, low mechanical strength, and unsuitability for a high-load operation due to low activity, and further improvement has been desired when these catalysts are used as industrial catalysts.

CITATION LIST

PATENT LITERATURE

Patent Literature 1: JP3710944B
Patent Literature 2: JP2017-176932A
Patent Literature 3: JP2003-10690A

SUMMARY OF INVENTION

TECHNICAL PROBLEM

An object of the present invention is to provide a catalyst with which an unsaturated carboxylic acid can be stably produced with excellent selectivity.

SOLUTION TO PROBLEM

As a result of intensive study to solve the above problems, the present inventors have found that a catalyst containing molybdenum, copper, and vanadium as essential components and having a ratio (X/Y) of a pore volume (X) of pores in a range of a pore diameter from 25 $\mu$m to 400 $\mu$m to a pore volume (Y) of pores in a range of a pore diameter from 0.1 $\mu$m to 4 $\mu$m, measured by mercury porosimetry, of 1.10 or more and 3.00 or less has high selectivity for an unsaturated carboxylic acid, and thus have completed the present invention.

That is, the present invention relates to the following 1) to 12).

1) A catalyst containing: molybdenum, copper, and vanadium as essential components, in which a ratio (X/Y) of a pore volume (X) of pores in a range of a pore diameter of 25 $\mu$m to 400 $\mu$m to a pore volume (Y) of pores in a range of a pore diameter of 0.1 $\mu$m to 4 $\mu$m, measured by mercury porosimetry, is 1.10 or more and 3.00 or less.

2) The catalyst according to the above 1), in which the X/Y is 1.15 or more and 2.50 or less.

3) The catalyst according to the above 1) or 2), in which the X/Y is 1.31 or more and 2.40 or less.

4) The catalyst according to any one of the above 1) to 3), further containing: arsenic as an essential component.

5) The catalyst according to any one of the above 1) to 4), in which a catalytically active component has a composition represented by the following formula (1):

$$Mo_{10}V_{a1}P_{b1}Cu_{c1}As_{d1}X_{e1}Y_{f1}O_{g1} \qquad (1),$$

where, Mo, V, P, Cu, As, and O represent molybdenum, vanadium, phosphorus, copper, arsenic, and oxygen respectively, X represents at least one element selected from the group consisting of Ag, Mg, Zn, Al, B, Ge, Sn, Pb, Ti, Zr, Sb, Cr, Re, Bi, W, Fe, Co, Ni, Ce, and Th, Y represents at least one element selected from the group consisting of K, Rb, Cs, and Tl, and a1, b1, c1, d1, e1, f1, and g1 each represent an atomic ratio of the element, $0.1 \le a1 \le 6$, $0 \le b1 \le 6$, $0 < c1 \le 3$, $0 < d1 < 3$, $0 \le e1 \le 3$, $0 \le f1 \le 3$, and g1 is a value determined according to a valence and an atomic ratio of other elements.

6) The catalyst according to the above 5), in which the catalytically active component having the composition represented by the (1) satisfies a relationship represented by the following expression (I):

$$0.6 \le a1/c1 \le 1.7 \qquad (I).$$

7) The catalyst according to the above 5) or 6), in which the catalytically active component having the composition represented by the (1) satisfies a relationship represented by the following expression (II):

$$-0.5 \le (a1-c1)/d1 \le 0.4 \qquad (II).$$

8) The catalyst according to any one of the above 1) to 7), in which the catalytically active component is carried on an inert carrier.

9) The catalyst according to the above 8), in which the inert carrier is silica and/or alumina.

10) The catalyst according to any one of the above 1) to 9), in which the catalyst is used for producing an unsaturated carboxylic acid compound.

11) A method for producing an unsaturated carboxylic acid compound using the catalyst according to any one of the above 1) to 10).

12) The production method according to the above 11), in which the unsaturated carboxylic acid compound is methacrylic acid.

ADVANTAGEOUS EFFECTS OF INVENTION

The present invention allows for providing a catalyst which contains molybdenum, copper, and vanadium as essential components and with which a desired product can be obtained with high selectivity. Therefore, in a catalytic gas phase oxidation reaction using the catalyst, the desired product can be obtained with higher selectivity and stability.

DESCRIPTION OF EMBODIMENTS

[About Catalyst (A)]

A catalyst according to the present invention is a composite oxide catalyst containing molybdenum, copper, and vanadium as essential components, in which a ratio (X/Y) of a pore volume (X) of pores in a range of a pore diameter from 25 $\mu$m to 400 $\mu$m to a pore volume (Y) of pores in a range of a pore diameter from 0.1 $\mu$m to 4 $\mu$m, measured by mercury porosimetry, is 1.10 or more and 3.00 or less. In the present description, the catalyst having the above configuration is referred to as a catalyst (A). In addition, in present description, "to" shall include numerical values that are before and after "to".

In the catalyst (A), the pore diameter and the pore volume can be measured by mercury porosimetry using a porosimeter. Here, the mercury porosimetry is a method of applying a pressure to mercury, which has a high surface tension, pressing mercury into pores or voids on a solid surface, determining a pore distribution based on a relationship between the pressure applied at this time and the volume of the pressed mercury, and determining the pore diameter and pore volume based on the pore distribution.Examples of the measurement method include a method of, by using a fully automatic pore distribution measuring apparatus (Pore Master 60-GT (Quanta Chrome Co.)) without subjecting the catalyst (A) to a pretreatment such as vacuum degassing, charging a sample having a weight of about 5 g to a large cell (10 mm in diameter × 6 cm) having a cell volume of 2 cc, setting the mercury surface tension to 480 dyn/cm and a mercury contact angle to 140°, performing measurement under conditions of a measurement temperature of 20°C and a pore diameter range for measurement of 0.0036 μm to 400 μm, assuming that all pores are cylindrical in measurement results, performing analysis using the pressure applied during measurement and the Washburn equation, and obtaining the pore distribution of each pore diameter of the catalyst.

A preferred lower limit of the (X/Y) is 1.10, 1.15, 1.20, 1.31, 1.35, 1.50, and 1.70 in order, and particularly preferably 1.90. The upper limit is 3.00, 2.90, 2.80, 2.70, 2.60, and 2.50 in order, and particularly preferably 2.40. Therefore, the most preferred range of the (X/Y) is 1.90 to 2.40. When the (X/Y) is less than 1.10 or more than 3.00, the optimum pore distribution for the reaction may disappear and the selectivity may decrease.

A preferred lower limit of the pore volume (X) of pores in a range of a pore diameter from 25 μm to 400 μm is 0.01, 0.02 and 0.03 in order, and particularly preferably 0.04. The upper limit is 0.08, 0.07, and 0.06 in order, and particularly preferably 0.05. Therefore, the most preferred range of the X is 0.04 to 0.05.

A preferred lower limit of the pore volume (Y) of pores in a range of a pore diameter from 1 μm to 4 μm is 0.015, 0.016, 0.017, and 0.018 in order, and particularly preferably 0.019. The upper limit is 0.040, 0.039, 0.038, 0.037, 0.036, 0.035, 0.034, 0.033, 0.032, and 0.031 in order, and particularly preferably 0.030. Therefore, the most preferred range of the X is 0.019 to 0.030.

Although it is not clear why satisfying this pore volume ratio allows for providing a desired product with high selectivity, it is considered that an optimum pore distribution for reaction prevents side reactions other than the oxidation reaction for obtaining a desired compound to stably obtain an unsaturated carboxylic acid compound, which is a final product, with high selectivity and high yield.

Examples of a method of adjusting the pore volume of the catalyst include changing the composition, adjusting a calcination time, a calcination atmosphere, and a binder amount and a binder type for shaping a slurry dried body, and mixing an organic aid. A method of changing the composition and a method of raising the calcination temperature or extending the calcination time are particularly effective.

For example, regarding the calcination temperature, even when the composition is the same, the (X/Y) can be increased by about 0.10 to 0.50 by increasing the calcination temperature by 10°C to 40°C. In addition, the (X/Y) can be increased by about 0.1 to 0.5 by shortening the calcination time by about 1 hour to 3 hours.

A preferred composition of a catalytically active component of the catalyst (A) is represented by the following general formula (1).

[Chem. 1]

$$Mo_{10}V_{a1}P_{b1}Cu_{c1}As_{d1}X_{e1}Y_{f1}O_{g1} \qquad (1)$$

Here, Mo, V, P, Cu, As, and O represent molybdenum, vanadium, phosphorus, copper, arsenic, and oxygen respectively. X represents at least one element selected from the group consisting of Ag (silver), Mg (magnesium), Zn (zinc), Al (aluminum), B (boron), Ge (germanium), Sn (tin), Pb (lead), Ti (titanium), Zr (zirconium), Sb (antimony), Cr (chromium), Re (rhenium), Bi (bismuth), W (tungsten), Fe (iron), Co (cobalt), Ni (nickel), Ce (cerium), and Th (thorium). Y represents at least one element selected from the group consisting of K (potassium), Rb (rubidium), Cs (cesium), and Tl (thallium). a1, b1, c1, d1, e1, f1, and g1 each represent an atomic ratio of the element, $0.1 \leq a1 \leq 6$, $0 \leq b1 \leq 6$, $0 < c1 \leq 3$, $0 < d1 < 3$, $0 \leq e1 \leq 3$, $0 \leq f1 \leq 3$, and g1 is a value determined according to a valence and an atomic ratio of other elements. The composition in the present invention means an active component, and silicon carbide, alumina, silica, silica-alumina, mullite, alundum, steatite, and the like can be used as an inert carrier.

In the composition of the above formula (1), X is preferably Zn, Ag, Fe, or Sb, more preferably Ag, Fe, or Sb, particularly preferably Fe or Sb, and most preferably Sb.

In the composition of the above formula (1), Y is preferably K, Rb, or Cs, more preferably K or Cs, and most preferably Cs, but a catalyst that do not contain the Y component tends to exhibit the effects of the present invention particularly remarkably.

In the composition of the above formula (1), preferred ranges of a1 to g1 are as follows.

The lower limit of a1 is 0.2, 0.25, 0.3, 0.35, 0.4, and 0.5 in order of preference, and most preferably 0.51. The upper limit of a1 is 5, 3, 2, 1, 0.8, 0.7, and 0.62 in order of preference, and most preferably 0.6. That is, the most preferred range of a1 is $0.51 \leq a1 \leq 0.6$.

The lower limit of b1 is 0, 0.1, 0.3, 0.5, 0.7, 0.9, and 1.0 in order of preference, and most preferably 1.05. The upper limit of b1 is 5, 4, 3, and 2 in order of preference, and most preferably 1.5. That is, the most preferred range of b1 is $1.05 \leq b1 \leq 1.5$.

The lower limit of c1 is 0.1, 0.2, and 0.3 in order of preference, and most preferably 0.4. The upper limit of c1 is 2, 1.5, 1.2, 1.0, and 0.8 in order of preference, and most preferably 0.6. That is, the most preferred range of c1 is $0.4 \leq c1 \leq 0.6$.

The lower limit of d1 is 0, 0.1, 0.2, 0.3, and 0.4 in order of preference, and most preferably 0.45. The upper limit of d1 is 2, 1.5, 1.2, 1.0, and 0.8 in order of preference, and most preferably 0.6. That is, the most preferred range of d1 is $0.45 \leq d1 \leq 0.6$.

The upper limit of e1 is 2, 1.5, 1, 0.5, 0.1, and 0.06 in order of preference, and most preferably 0.065. The lower limit of e1 is 0, and X being not contained, that is, e1 = 0, is the most preferred composition of the catalyst (A).

The upper limit of f1 is 2, 1.5, 1, 0.5, and 0.1 in order of preference, and most preferably 0.05. The lower limit of f1 is 0, and Y being not contained, that is, f1 = 0, is the most preferred composition of the catalyst (A).

In the formula (1), when the relationship between a1 and c1 satisfies the above expression (I), the catalyst (A) has a particularly preferred catalyst composition.

The upper limit of al/cl is 1.65, 1.6, 1.55, 1.5, 1.45, 1.4, and 1.35 in order of preference, and particularly preferably 1.3. The lower limit is 0.65, 0.7, 0.75, and 0.8 in order of preference, and particularly preferably 0.85. Therefore, the most preferred range of the al/cl is $0.85 \leq a1/c1 \leq 1.3$.

In the formula (1), when the relationship between a1, c1, and d1 satisfies the above expression (II), the catalyst (A) has a particularly preferred catalyst composition.

The upper limit of (al-cl)/dl is 0.40, 0.38, and 0.37 in order of preference, and particularly preferably 0.36 and 0.35, and most preferably 0.34. The lower limit is -0.48, -0.46, -0.44, -0.42, -0.40, and -0.38 in order of preference, and particularly preferably -0.36. Therefore, the most preferred range of the (al-cl)/dl is $-0.36 \leq (a1-c1)/d1 \leq 0.34$.

[Method for Producing Catalyst (A)]

A method for producing the catalyst (A) includes: (a) a step of dispersing compounds containing each or a plurality of the above metals in water to prepare an aqueous solution or aqueous dispersion of the compounds (hereinafter both are referred to as a slurry liquid); (b) a step of drying the slurry liquid obtained in the step (a) to obtain a slurry dried body; (c) a step of shaping the slurry dried body obtained in the step (b); and (d) a step of calcining a coated shaped product obtained in the step (c). Hereinafter, preferred embodiments are described for each step, but implementation of the present invention is not limited to the following embodiments.

The step (a) includes a step of preparing compounds containing active component elements and a step of mixing the compounds with water.

In the step (a), compounds containing essential active component elements of the catalyst according to the present invention and optional active component elements are used. Examples of the compounds include a chloride, a sulfate, a nitrate, an oxide or an acetate of the active component elements. More specifically, preferred examples of the compounds include, but not limited to, nitrates such as cobalt nitrate, acetates such as copper acetate, oxides such as molybdenum oxide, vanadium pentoxide, copper oxide, antimony trioxide, cerium oxide, zinc oxide, or germanium oxide, and acids (or salts thereof) such as orthophosphoric acid, phosphoric acid, boric acid, aluminum phosphate, or 12-tungstophosphoric acid. The compound containing these active components may be used alone or in combination of two or more thereof. In the step (a), the compounds containing each active component and water are uniformly mixed to obtain a slurry liquid. In the slurry liquid, not all the components need to be dissolved in water, and a part or the whole thereof may be in a suspended state. The amount of water to be used in the slurry liquid is not limited as long as it can completely dissolve the entire amount of the compounds to be used or can enable uniform mixing. The amount of water to be used may be appropriately determined in consideration of the drying method and drying conditions in the step (b). Basically, the amount of water is about 200 to 2000 parts with respect to 100 parts of the total mass of the compounds for slurry liquid preparation. The amount of water may be large, but when it is too large, the energy cost in the drying step, i.e., the step (b) increases, and this may have many disadvantages such as being unable to dry completely.

In the present invention, the shape of stirring blades of a stirrer to be used in the step (a) is not limited, and any stirring blades such propeller blades, turbine blades, paddle blades, inclined paddle blades, screw blades, anchor blades, ribbon blades, and large lattice blades can be used in one stage or two or more stages of the same type of blade or different types of blades in the up-down direction. In addition, a baffle (baffle plate) may be installed in a reaction vessel as required.

In the step (b), the slurry liquid obtained in the step (a) is completely dried. The drying method is not limited, but examples thereof include drum drying, freeze drying, spray drying, and evaporation to dryness. Among these, in the present invention, spray drying is preferred because it allows for drying the slurry liquid into a powder or granules in a short period of time. The drying temperature in the spray drying varies depending on the concentration of the slurry liquid, the liquid feeding rate, or the like, and the temperature at the outlet of the dryer is typically 70°C to 150°C.

The step (c) includes a step of calcination the slurry dried body obtained in the step (b) (this step is not essential), a step of mixing the slurry dried body with an additive, and a step of shaping the slurry dried body or a mixture of the slurry dried body and the additive.

In the step (c), the slurry dried body obtained in the step (b) is shaped. When the slurry dried body is calcined at about 250°C to 350°C and then shaped, the mechanical strength or the catalyst performance may be improved, so that the slurry dried body may be calcined before shaping. The shaping method is not limited, and in order to reduce the pressure loss of a reaction gas in an oxidation reaction, the slurry dried body may be shaped into a column, tablet, ring, sphere, or the like, or an inert carrier may be coated with the slurry dried body. Among these, coating an inert carrier with the slurry dried body to obtain a coated catalyst is preferable, since it can be expected to improve selectivity and remove reaction heat. This coating step is preferably a tumbling granulation method described below. This method is a method of rotating a flat or uneven disc at a high speed in an apparatus that has the disc at, for example, the bottom in a fixed container, to vigorously stirring a carrier in the container by repeating rotation and revolution, and coating the carrier with a coating mixture obtained by adding a binder, the slurry dried body obtained in the step (b), and, if necessary, other additives such as a shaping aid and a strength improver thereto. As a method for adding the binder, any method may be used, such as 1) pre-mixing the binder with the coating mixture, 2) adding the binder simultaneously with adding of the coating mixture into the fixed container, 3) adding the binder after adding of the coating mixture into the fixed container, 4) adding the binder before adding of the coating mixture into the fixed container, and 5) separating each of the coating mixture and the binder and adding the total amount thereof by appropriately combining 2) to 4). Among these, in 5), it is preferable to adjust an addition rate by using an auto feeder or the like such that a predetermined amount of the coating mixture is carried on the carrier without, for example, the coating mixture adhering to walls of the fixed container or agglomeration of the coating mixture. The binder is preferably water, or at least one selected from the group consisting of organic compounds having a boiling point of 150°C or lower at 1 atm or less, or an aqueous solution thereof. Specific examples of the binder other than water include alcohols such as methanol, ethanol, propanol, or butanol, preferably an alcohol having 1 to 4 carbon atoms, ethers such as ethyl ether, butyl ether, or dioxane, esters such as ethyl acetate or butyl acetate, ketones such as acetone or methyl ethyl ketone, and an aqueous solution thereof. Ethanol is particularly preferred. When ethanol is used as the binder, ethanol/water is preferably 10/0 to 0/10 (mass ratio), and preferably 9/1 to 1/9 (mass ratio) when mixed with water. The amount of the binder to be used is typically 2 to 60 parts by mass, and preferably 10 to 50 parts by mass, with respect to 100 parts by mass of the coating mixture.

Specific examples of the inert carrier in the above coating include silicon carbide, alumina, silica, silica-alumina, mullite, alundum, and steatite. Silicon carbide, alumina, silica, silica-alumina, and steatite are preferred, and alumina, silica, and silica-alumina are more preferred. The diameter of the carrier is, for example, 1 mm to 15 mm. Preferably, a spherical carrier having a diameter of 2.5 mm to 10 mm, and the like are included. The component in the carrier is preferably 90 mass% or more, and more preferably 95 mass% or more. The carrier having a porosity of 10% to 70% are typically used. The proportion of the carrier with respect to the coating mixture is typically, coating mixture/(coating mixture + carrier) = 10 mass% to 75 mass%. The proportion of 15 mass% to 60 mass% is preferable. When the proportion of the coating mixture is large, the reaction activity of the coated catalyst tends to increase, but the mechanical strength tends to decrease. Conversely, when the proportion of the coating mixture is small, the mechanical strength tends to increase, but the reaction activity tends to decrease. Examples of the shaping aid to be used if necessary in the above include silica gel, diatomaceous earth, and an alumina powder. The amount of the shaping aid to be used is typically 1 to 60 parts by mass with respect to 100 parts by mass of a solid of the catalytically active component. In addition, if necessary, inorganic fibers (for example, ceramic fibers, or whiskers) inert to the catalytically active component and a reaction gas are useful as a strength improver for improving the mechanical strength of the catalyst. The amount of the fiber to be used is typically 1 to 30 parts by mass with respect to 100 parts by mass of a solid of the catalytically active component. In addition, the inert carrier in the present invention is a carrier that does not have activity on the raw material and the product, and, for example, has a methacrolein conversion rate of 3.0% or less under typically known reaction conditions.

In the step (d), the shaped dried body or coated catalyst of the step (b) obtained in step (c) is calcined. The dried body or coated catalyst can be directly used as a catalyst for the catalytic gas phase oxidation reaction, but calcination is preferred because calcination stabilizes the structure and improves the catalyst performance. When the calcination temperature is too high, the heteropoly acid may decompose and the catalyst performance may decrease. Therefore, the calcination temperature is typically 100°C to 400°C, preferably 250°C to 380°C, more preferably 270°C to 360°C, and particularly preferably 290°C to 340°C. When the calcination time is too short, the structure of the heteropoly acid may become unstable and the catalyst performance may decrease. When the calcination time is too long, the production efficiency of the catalyst decreases. The calcination time is typically 1 hour to 20 hours. The calcination is typically performed in an air atmosphere, or may be performed in an inert gas atmosphere such as nitrogen or a reducing gas atmosphere such as ethanol. After the calcination in an inert gas or reducing gas atmosphere, the calcination may be further performed in an air atmosphere if necessary. The proportion of the active component to the entire coated catalyst after the calcination obtained as described above is 10 mass% to 60 mass%.

The catalyst according to the present invention obtained by the method for producing the catalyst according to the present invention is used in a reaction in which an unsaturated aldehyde is catalytically oxidized in a gas phase with molecular oxygen to obtain an unsaturated carboxylic acid. Among these, it is suitably used for the production of methacrylic acid by catalytic gas phase oxidation of methacrolein.

Molecular oxygen or a molecular oxygen-containing gas is used in the catalytic gas phase oxidation reaction.

The proportion of the molecular oxygen with respect to an unsaturated aldehyde such as methacrolein is preferably in the range of 0.5 to 20, and more preferably in the range of 1 to 10 in terms of molar ratio. For example, for the purpose of allowing the reaction to proceed smoothly, it is preferable to add water to the raw material gas in a range of 1 to 20 with respect to methacrolein in terms of molar ratio. The raw material gas may contain oxygen, if necessary, water (typically contained as water vapor), as well as gases inert to the reaction, such as nitrogen, a carbon dioxide gas, and saturated hydrocarbons.

As unsaturated aldehyde of the raw material, a gas containing an unsaturated aldehyde obtained by oxidizing an alkene compound, alcohol compound or ether compound, which are raw materials, may be used as it is. In the case of methacrolein, a methacrolein-containing gas obtained by oxidizing isobutylene, tertiary butanol, and methyl tertiary butyl ether may be supplied as it is.

In the catalytic gas phase oxidation reaction, the reaction temperature is typically 200°C to 400°C, and preferably 260°C to 360°C, and the supply rate of the raw material gas is typically 100 hr$^{-1}$ to 6000 hr$^{-1}$, and preferably 300 hr$^{-1}$ to 3000 hr$^{-1}$ in terms of space velocity (SV). In addition, the catalytic gas phase oxidation reaction can be carried out under a pressure or under a reduced pressure, and a pressure near an atmospheric pressure is typically suitable.

When producing an unsaturated carboxylic acid using the catalyst according to the present invention, the catalyst for producing the corresponding unsaturated aldehyde using propylene, isobutylene, t-butyl alcohol, etc. as raw materials is not limited. For example, it is preferable to use a composite oxide catalyst containing molybdenum and bismuth described in Patent Literature 4.

EXAMPLES

The present invention will be described in more detail with reference to Examples below, but the present invention is not limited to these Examples. In Examples, "parts" means parts by weight, and "%" means wt%.

[Example 1]

1) Preparation of Catalyst

To 7100 parts of pure water, 1000 parts of molybdenum oxide, 37.91 parts of vanadium pentoxide, 22.11 parts of cupric oxide, 88.08 parts of a 85% phosphoric acid aqueous solution, and 98.60 parts of a 60% arsenic acid aqueous solution were added, and the mixture was heated and stirred at 92°C for 10 hours to obtain a reddish brown transparent solution. Subsequently, this solution was spray-dried to obtain a slurry dried body. The composition of the solid of the catalytically active component obtained from the charged amount of raw materials is Mo-10, V-0.6, P-1.1, As-0.6, and Cu-0.4.

Next, 214 parts of the slurry dried body and 29.8 parts of a strength improving material (alumina-silica fibers) were uniformly mixed, and the mixture was coated and shaped onto 200 parts of a spherical porous alumina carrier (particle size: 3.5 mm), with about 30 parts of a 90% ethanol aqueous solution as a binder. Next, the obtained shaped product was calcined at 310°C for 6 hours under air circulation to obtain a catalyst (coated catalyst) according to the present invention.

2) Production of Methacrylic Acid

A stainless reaction tube having an inner diameter of 18.4 mm was filled with 40.2 ml of the obtained coated catalyst according to the present invention, and an oxidation reaction of methacrolein was carried out under the conditions of a raw material gas (composition (molar ratio); methacrolein: oxygen: water vapor: nitrogen = 1:2:4: 18.6) and a space velocity (SV) of 900 hr$^{-1}$. The reaction bath temperature was adjusted between 290°C and 330°C and the methacrylic acid selectivity at a methacrolein conversion rate of 77 mol % was calculated.

The conversion rate and the selectivity are defined as follows.

Conversion rate = number of moles of reacted methacrolein/number of moles of supplied methacrolein × 100

Selectivity = number of moles of methacrylic acid produced/number of moles of methacrolein reacted × 100

3) Pore Distribution Measurement

The pore distribution of the obtained catalyst was measured using the following apparatus and conditions. Apparatus used: fully automatic pore distribution measuring apparatus Pore Master 60GT (Quanta Chrome Co.)

Sample weight: 1 g to 5 g
Pretreatment on sample: none
Sample cell: Large cell (10 mm in diameter × 6 cm)
Measurement range: all ranges
Measurement range: pore diameter 400 μm to 0.0036 μm
Calculation range: pore diameter 400 μm to 0.0036 μm
Mercury contact angle: 140 deg
Mercury surface tension: 480 dyn/cm
Measurement cell volume: 2.0 cc

Pore distribution measurement was performed under the above conditions, and the ratio (X/Y) of the pore volume (X) of pores in a range of a pore diameter from 25 μm to 400 μm to the pore volume (Y) of pores in a range of a pore diameter from 0.1 μm to 4 μm was calculated.

[Example 2]

1) Preparation of Catalyst

To 7100 parts of pure water, 1000 parts of molybdenum oxide, 39.17 parts of vanadium pentoxide, 24.87 parts of cupric oxide, 93.69 parts of a 85% phosphoric acid aqueous solution, and 82.16 parts of a 60% arsenic acid aqueous solution were added, and the mixture was heated and stirred at 92°C for 10 hours to obtain a reddish brown transparent solution. Subsequently, 7.09 parts of antimony trioxide was added to this solution, and the mixture was heated and stirred for 4 hours to obtain a dark green transparent solution. Subsequently, this solution was spray-dried to obtain a slurry dried body. The composition of the solid of the catalytically active component obtained from the charged amount of raw materials is Mo-10, V-0.6, P-1.2, As-0.5, Cu-0.5, and Sb-0.1.
Next, 214 parts of the slurry dried body and 29.8 parts of a strength improving material (alumina-silica fibers) were uniformly mixed, and the mixture was coated and shaped onto 200 parts of a spherical porous alumina carrier (particle size: 3.5 mm), with about 30 parts of a 90% ethanol aqueous solution as a binder. Next, the obtained shaped product was calcined at 310°C for 4 hours under air circulation to obtain a catalyst (coated catalyst) according to the present invention.

[Example 3]

A catalyst was prepared in the same manner as in Example 2, except that 39.91 parts of vanadium pentoxide was set to 37.91 parts, 24.87 parts of cupric oxide was set to 22.11 parts, 93.69 parts of a 85% phosphoric acid aqueous solution was set to 88.08 parts, 82.16 parts of a 60% arsenic acid aqueous solution was set to 98.60 parts, 7.09 parts of antimony trioxide was set to 5.06 parts, and the calcination time was changed from 4 hours to 6 hours. The composition of the solid of the catalytically active component obtained from the charged amount of raw materials is Mo-10, V-0.6, P-1.1, As-0.6, Cu-0.4, and Sb-0.1.

[Example 4]

A catalyst was prepared in the same manner as in Example 2, except that 39.91 parts of vanadium pentoxide was set to 37.91 parts, 24.87 parts of cupric oxide was set to 22.11 parts, 93.69 parts of a 85% phosphoric acid aqueous solution was set to 96.09 parts, and 7.09 parts of antimony trioxide was set to 5.06 parts. The composition of the solid of the catalytically active component obtained from the charged amount of raw materials is Mo-10, V-0.6, P-1.2, As-0.5, Cu-0.4, and Sb-0.1.

[Example 5]

A catalyst was prepared in the same manner as in Example 1, except that the calcination time was changed from 4 hours to 6 hours.

[Example 6]

A catalyst was prepared in the same manner as in Example 2, except that the calcination time was changed from 4 hours to 6 hours.

[Comparative Example 1]

A catalyst was prepared in the same manner as in Example 1, except that 37.91 parts of vanadium pentoxide was set to 44.23 parts, 22.11 parts of cupric oxide was set to 11.05 parts, and 98.60 parts of a 60% arsenic acid aqueous solution was set to 82.16 parts. The composition of the solid of the catalytically active component obtained from the charged amount of raw materials is Mo-10, V-0.7, P-1.1, As-0.5, and Cu-0.2.

[Comparative Example 2]

A catalyst was prepared in the same manner as in Example 2, except that 39.91 parts of vanadium pentoxide was set to 31.59 parts, 24.87 parts of cupric oxide was set to 11.05 parts, 93.69 parts of a 85% phosphoric acid aqueous solution was set to 80.07 parts, and 7.09 parts of antimony trioxide was set to 10.13 parts. The composition of the solid of the catalytically active component obtained from the charged amount of raw materials is Mo-10, V-0.5, P-1.0, As-0.5, Cu-0.2, and Sb-0.1.

According to the same method as in Example 1, production of methacrylic acid using the catalysts in Examples 2 to 6 and Comparative Examples 1 and 2 was performed, and the pore distribution measurement of the catalysts was performed. These results are shown in Table 1 together with the results for the catalyst in Example 1.

[Table 1]

|  | Methacrylic acid selectivity (mol%) | X/Y | X | Y |
|---|---|---|---|---|
| Example 1 | 88.5 | 2.35 | 0.047 | 0.020 |
| Example 2 | 88.6 | 1.86 | 0.041 | 0.022 |
| Example 3 | 88.0 | 1.30 | 0.026 | 0.020 |
| Example 4 | 87.5 | 1.11 | 0.039 | 0.035 |
| Example 5 | 88.8 | 1.89 | 0.036 | 0.019 |
| Example 6 | 88.2 | 1.65 | 0.043 | 0.026 |
| Comparative Example 1 | 86.2 | 0.87 | 0.034 | 0.039 |
| Comparative Example 2 | 86.2 | 1.05 | 0.046 | 0.044 |

As is clear from Table 1, the catalysts according to the present invention in Examples have methacrylic acid selectivity higher than that of the catalysts in Comparative Examples.

Although the present invention has been described in detail with reference to specific embodiments, it will be apparent to those skilled in the art that various changes and modifications can be made without departing from the spirit and scope of the present invention.

This application is based on a Japanese patent application (Japanese Patent Application No. 2021-10889) filed on January 27, 2021, the entirety of which is incorporated by reference. In addition, all references cited herein are incorporated in entirety thereof.

INDUSTRIAL APPLICABILITY

The present invention provides a catalyst which contains molybdenum, copper, and vanadium as essential components and with which a desired product can be obtained with high selectivity. Therefore, in a catalytic gas phase oxidation reaction using the catalyst, the desired product can be obtained with higher selectivity, yield, and stability.

Claims

1. A catalyst comprising: molybdenum, copper, and vanadium as essential components, wherein a ratio (X/Y) of a pore volume (X) of pores in a range of a pore diameter from 25 $\mu$m to 400 $\mu$m to a pore volume (Y) of pores in a range of a pore diameter of 0.1 $\mu$m to 4 $\mu$m, measured by mercury porosimetry, is 1.10 or more and 3.00 or less.

2. The catalyst according to claim 1, wherein the X/Y is 1.15 or more and 2.50 or less.

3. The catalyst according to claim 1 or 2, wherein the X/Y is 1.31 or more and 2.40 or less.

4. The catalyst according to any one of claims 1 to 3, further comprising: arsenic as an essential component.

5. The catalyst according to any one of claims 1 to 4, wherein a catalytically active component has a composition represented by the following formula (1):

$$Mo_{10}V_{a1}P_{b1}Cu_{c1}As_{d1}X_{e1}Y_{f1}O_{g1} \qquad (1),$$

where, Mo, V, P, Cu, As, and O represent molybdenum, vanadium, phosphorus, copper, arsenic, and oxygen respectively, X represents at least one element selected from the group consisting of Ag, Mg, Zn, Al, B, Ge, Sn, Pb, Ti, Zr, Sb, Cr, Re, Bi, W, Fe, Co, Ni, Ce, and Th, Y represents at least one element selected from the group consisting of K, Rb, Cs, and Tl, and a1, b1, c1, d1, e1, f1, and g1 each represent an atomic ratio of the element, $0.1 \le a1 \le 6$, $0 \le b1 \le 6$, $0 < c1 \le 3$, $0 < d1 < 3$, $0 \le e1 \le 3$, $0 \le f1 \le 3$, and g1 is a value determined according to a valence and an atomic ratio of other elements.

6. The catalyst according to claim 5, wherein the catalytically active component having the composition represented by the (1) satisfies a relationship represented by the following expression (I):

$$0.6 \le a1/c1 \le 1.7 \qquad (I).$$

7. The catalyst according to claim 5 or 6, wherein the catalytically active component having the composition represented by the (1) satisfies a relationship represented by the following expression (II):

$$-0.5 \le (a1-c1)/d1 \le 0.4 \qquad (II).$$

8. The catalyst according to any one of claims 1 to 7, wherein the catalytically active component is carried on an inert carrier.

9. The catalyst according to claim 8, wherein the inert carrier is silica and/or alumina.

10. The catalyst according to any one of claims 1 to 9, wherein the catalyst is used for producing an unsaturated carboxylic acid compound.

11. A method for producing an unsaturated carboxylic acid compound using the catalyst according to any one of claims 1 to 10.

12. The production method according to claim 11, wherein the unsaturated carboxylic acid compound is methacrylic acid.

## INTERNATIONAL SEARCH REPORT

| International application No. |
|---|
| **PCT/JP2022/002960** |

**A.  CLASSIFICATION OF SUBJECT MATTER**

*B01J 27/199*(2006.01)i; *C07B 61/00*(2006.01)i; *C07C 51/235*(2006.01)i; *C07C 57/055*(2006.01)i
FI:  B01J27/199 Z; C07B61/00 300; C07C51/235; C07C57/055 B

According to International Patent Classification (IPC) or to both national classification and IPC

**B.  FIELDS SEARCHED**

Minimum documentation searched (classification system followed by classification symbols)

B01J27/199; C07B61/00; C07C51/235; C07C57/055

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| A | JP 2020-157294 A (NIPPON KAYAKU CO., LTD.) 01 October 2020 (2020-10-01) entire text | 1-12 |
| A | WO 2015/053269 A1 (NIPPON KAYAKU CO., LTD.) 16 April 2015 (2015-04-16) entire text | 1-12 |
| A | JP 2017-176932 A (MITSUBISHI CHEMICAL CORP.) 05 October 2017 (2017-10-05) entire text | 1-12 |
| A | JP 2000-024502 A (MITSUI CHEMICALS, INC.) 25 January 2000 (2000-01-25) entire text | 1-12 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **22 March 2022** | **05 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2022/002960**

| Patent document cited in search report | | | Publication date (day/month/year) | Patent family member(s) | Publication date (day/month/year) |
|---|---|---|---|---|---|
| JP | 2020-157294 | A | 01 October 2020 | (Family: none) | |
| WO | 2015/053269 | A1 | 16 April 2015 | US 2016/0244393 A1 entire text<br>EP 3056482 A1<br>CN 105612141 A<br>KR 10-2016-0068763 A | |
| JP | 2017-176932 | A | 05 October 2017 | (Family: none) | |
| JP | 2000-024502 | A | 25 January 2000 | (Family: none) | |

Form PCT/ISA/210 (patent family annex) (January 2015)

**EP 4 286 049 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3710944 B
- JP 2017176932 A
- JP 2003010690 A
- JP 2021010889 A